# EUROPÄISCHE PATENTSCHRIFT

(11) **EP 3 993 731 B1**
(45) Veröffentlichungstag und Bekanntmachung des Hinweises auf die Patenterteilung: **26.07.2023**
(21) Anmeldenummer: 20750575.1
(22) Anmeldetag: 29.06.2020
(51) Int. Cl.: A61C 8/02, A61F 2/28

(54) **ABDECKVORRICHTUNG FÜR EINE KNOCHENDEFEKTSTELLE UND VERFAHREN ZUR HERSTELLUNG EINER ABDECKVORRICHTUNG FÜR EINE KNOCHENDEFEKTSTELLE**
COVERING DEVICE FOR A BONE DEFECT AND METHOD FOR PRODUCING A COVERING DEVICE FOR A BONE DEFECT
DISPOSITIF DE RECOUVREMENT POUR UN DÉFAUT OSSEUX ET PROCÉDÉ DE FABRICATION D'UN DISPOSITIF DE RECOUVREMENT POUR UN DÉFAUT OSSEUX

(30) Priorität: 04.07.2019 DE 102019118134
(43) Veröffentlichungstag der Anmeldung: 11.05.2022
(73) Patentinhaber: Reoss GmbH, 70794 Filderstadt (DE)
(72) Erfinder: SEILER, Marcus, 70597 Stuttgart (DE)
(74) Vertreter: Patentanwälte Schuster, Müller & Partner mbB
(86) Internationale Anmeldenummer: PCT/DE2020/100562
(87) Internationale Veröffentlichungsnummer: WO 2021/000995

(56) Entgegenhaltungen:
- DE-A1- 4 226 465
- DE-A1- 4 302 708
- DE-A1-102016 000 236
- DE-C2- 4 226 465
- DE-C2- 4 302 708
- DE-U1-202015 005 610

## Beschreibung

### Stand der Technik

Die Erfindung geht aus von einem Verfahren zur Herstellung einer Abdeckvorrichtung für eine Knochendefektstelle, nach der Gattung des Anspruchs 1, und einer Abdeckvorrichtung für eine Knochendefektstelle, nach der Gattung des Anspruchs 15.

Knochendefektstellen in Form von Ausnehmungen oder Höhlungen im körpereigenen Knochengewebe werden in der Knochenchirurgie, beispielsweise bei der Rekonstruktion von Knochen in der orthopädischen, neurochirurgischen oder plastischen Chirurgie oder bei kieferchirurgischen Operationen, oftmals mit Knochenaufbaumaterial gefüllt. In der Regel besteht das Knochenaufbaumaterial aus einer Mischung aus synthetischen Knochenersatzmaterial (z.B. Hydroxylapatitgranulat) und körpereigenen Knochenpartikeln. Damit das Knochenaufbaumaterial im Wesentlichen ausschließlich von der Knochenseite her knöchern durchwachsen wird, wird die Ausnehmung, wie in der Patentschrift DE 43 02 708 C2 beschrieben, mit einer Abdeckmembran verschlossen. Befestigt wird die Abdeckmembran mit Befestigungsnägeln am körpereigenen, an die Knochendefektstelle angrenzenden gesunden Knochen, der durch die Befestigungsnägel beschädigt wird, wobei, da die Abdeckmembran aus einem flexiblen Material besteht, die Befestigung ein Höchstmaß an handwerklichem Geschick des Chirurgen erfordert.

Um diesen Nachteil einer fehlenden Stützfunktion der Abdeckmembran zu überwinden, wird in der Patentschrift US 48 16 339 eine Abdeckmembran beschrieben, die aus mehreren Schichten besteht, wobei diese Schichten nicht aus resorbierbaren Membranmaterial bestehen. Dabei ist es gegebenenfalls erforderlich, dass nach dem Ausheilen des Knochendefektes ein zweiter Eingriff notwendig ist, um körperfremdes Material zu entfernen.

In der Patentschrift DE 10 2005 039 382 B4 wird ein biodegradierbarer Hohlkörper, der insbesondere eine hohlzylindrische oder kegelzylindrische Form aufweist, vorgeschlagen. Der Hohlkörper weist in seinen Wandungen eine Mehrzahl von Öffnungen auf, durch die eine Aufnahme von Blut und damit der Aufbau von Eigenknochen möglich ist. Nachteilig ist hierbei, dass zum Einsetzen des Hohlkörpers eine zylindrische Bohrung mittels eines Bohrers in den vorhandenen Knochen eingebracht werden muss.

In der Offenlegungsschrift DE 10 2006 047 054 A1 wird ein Implantatlager vorgeschlagen, das sich durch eine hohe Passgenauigkeit und Stabilität auszeichnet, so dass der behandelnde Arzt es einfach handhaben und implantieren kann. Das aus Hydroxylapatit angefertigte Implantatlager, das zum Schutz der Schleimhaut vor mechanischen Einwirkungen und zum Schutz des Implantatlagers vor einwachsenden Gewebe von Seiten der Schleimhaut auf der der Schleimhaut zugewandten Seite eine dünne, insbesondere aus resorbierbaren Material bestehende, Membran aufweist, wird mit einem aufbauenden Fertigungsverfahren hergestellt, so dass die Materialbeschaffenheit eine "Gradientenstruktur" im Sinne einer insbesondere nach innen abnehmenden Dichte bildet. Dabei ist an der dem Knochen zugewandten Seite eine Bauweise mit einer insbesondere porösen Struktur und an der Außenseite des Implantatlagers, an der sich eine Struktur zur Halterung eines Zahnimplantats und/oder eines Zahnersatzes befindet, eine kompakte Bauweise vorgesehen.

Des Weiteren werden in den Offenlegungsschriften DE 198 30 992 A1, DE 10 2005 060 761 A1, DE 42 26 465 A1, WO 01/91818 A1, DE 10 2005 041 412 A1, DE 10 2006 047 054 A1, US 2011/0151400 A1, WO 00/59409 A1, WO 96/12446 A1, EP 2 737 871 A2 und WO 2006/051401 A2, sowie der Gebrauchsmusterschrift DE 20 2015 005 610 U1 und der Patentschrift US 7,172,422 B1 Vorrichtungen für eine Knochendefektstelle beschrieben, wobei sämtliche dieser Lösungen den Nachteil aufweisen, dass sie den neben der Knochendefektstelle vorhandenen gesunden Knochen in Mitleidenschaft ziehen.

Um diesen Nachteil zu vermeiden, wird in den Offenlegungsschriften DE 10 2011 011 191 A1 und DE 10 2016 000 236 A1 eine passgenaue Abdeckvorrichtung vorgeschlagen, die aber gerade durch die Passgenauigkeit den Nachteil aufweist, dass deren Positionierung an der Knochendefektstelle erschwert ist.

Um diesen Nachteil zu vermeiden, wird in der Offenlegungsschrift DE 10 2015 006 154 A1 eine Abdeckvorrichtung vorgeschlagen, die einem Aufsatz, der eine dem Knochendefekt abgewandte Seite und eine dem Knochendefekt zugewandte Seite und ggfls. mindestens ein Fixiermittel zur Fixierung des Aufsatzes an einem Knochen aufweist, wobei der Aufsatz aus einem formstabilen Material besteht, das zumindest teilweise mit dem Knochen in Berührung steht, und eine dem Knochendefekt zugewandte Seite des Aufsatzes oder eine dem Knochendefekt abgewandte Seite des Aufsatzes der Form des regenerierten Knochens entspricht, wobei der Aufsatz mindestens ein Positioniermittel aufweist. Zwar wir durch das Positioniermittel die Positionierung des Aufsatzes in einer Einsetzposition erleichtert, doch wird dadurch nicht das Problem gelöst, dass die Fixierung des Aufsatzes mittels eines Fixiermittels eventuell ein Höchstmaß an handwerklichem Geschick des Chirurgen erfordert.

### Die Erfindung und ihre Vorteile

Das erfindungsgemäße Verfahren zur Herstellung einer Abdeckvorrichtung für eine Knochendefektstelle, wobei mit dem Begriff "Knochendefektstelle" eine Stelle eines (kranken, deformierten, verletzten, durch Alterungsprozesse veränderten, durch Degeneration (z.B. nach Zahnextraktion, Tumor usw.) veränderten und/oder im Volumen veränderten) Knochens (z.B. Hüfte, Wirbelsäule, Kopf, Kiefer odgl.) eines Menschen oder eines Tieres bezeichnet wird, die von der Form und/oder dem Volumen eines gesunden Knochens abweicht, mit den Merkmalen des Anspruchs 1, und die erfindungsgemäße Abdeckvorrichtung für eine Knochendefektstelle, mit den Merkmalen des Anspruchs 15, haben demgegenüber den Vorteil, dass die Abdeckvorrichtung einen Aufsatz (z.B. Formschale, starre Schale, Formkörper), der ein- oder mehrschichtig ausgestaltet sein kann, und mindestens ein Fixiermittel zur Fixierung des Aufsatzes an einem Knochen besteht, wobei an dem Aufsatz mindestens ein zur Fixierung des Aufsatzes geeignetes Fixiermittel mittels mindestens einer Verbindung lösbar angeordnet ist, wodurch ein Einsatz der Abdeckvorrichtung sehr erleichtert wird.

Das erfindungsgemäße Verfahren zur Herstellung einer Abdeckvorrichtung für eine Knochendefektstelle, die einen Aufsatzes und mindestens ein Fixiermittel für den Aufsatz aufweist, besteht aus folgenden Verfahrensschritten:
- Aufnahme eines ersten Datensatzes, der die betroffene Knochendefektstelle im Ist-Zustand repräsentiert,
- Vergleich des ersten Datensatzes mit einem zweiten Datensatz, der den Soll-Zustand eines an der Knochendefektstelle regenerierten Knochens repräsentiert,
- Verwendung des ersten Datensatzes und des zweiten Datensatzes zur Planung des Aufsatzes,
   - der dazu geeignet ist, die Knochendefektstelle zumindest teilweise abzudecken, wobei der Aufsatz eine der Knochendefektstelle abgewandte Seite und eine der Knochendefektstelle zugewandte Seite aufweist und der Aufsatz mit mindestens einem Fixiermittel innerhalb der Knochendefektstelle fixierbar ist,
      oder
   - der dazu geeignet ist, die Knochendefektstelle zumindest teilweise abzudecken und zusätzlich einen an die Knochendefektstelle angrenzenden gesunden Knochen zumindest teilweise abzudecken, wobei der Aufsatz eine dem gesunden Knochen abgewandte Seite und eine der Knochendefektstelle abgewandte Seite und eine dem gesunden Knochen zugewandte Seite und eine der Knochendefektstelle zugewandte Seite aufweist und der Aufsatz mit mindestens einem Fixiermittel innerhalb der Knochendefektstelle und/oder mit mindestens einem Fixiermittel an dem gesunden Knochen fixierbar ist,
- Umsetzung der Planung des Aufsatzes in einen Planungsdatensatz und
- Zuführung des Planungsdatensatzes an ein Fertigungsverfahren, insbesondere an ein computergesteuertes Fertigungsverfahren, wobei während und/oder nach der Fertigung des Aufsatzes (4) mindestens ein zur Fixierung des Aufsatzes innerhalb der Knochendefektstelle oder an dem an die Knochendefektstelle angrenzenden gesunden Knochen geeignetes Fixiermittel mittels mindestens einer zwischen dem Fixiermittel und dem Aufsatz angeordneten Verbindung lösbar mit dem Aufsatz verbunden wird, wobei die Lösung mindestens einer Verbindung irreversibel ist und mindestens eine Verbindung eine Sollbruchstelle ist.

Bevorzugt wird bei der Herstellung der Abdeckvorrichtung eine rechnergestützte Formgebung (CAD) des Aufsatzes mit einer rechnergestützten Fertigung (CAM) zu CAD/CAM kombiniert, so dass ein am Computer entwickeltes Entwurfsmodell des Aufsatzes direkt elektronisch an die Fertigung übermittelt werden kann.

Nach einer vorteilhaften Ausgestaltung des erfindungsgemäßen Verfahrens ist während mindestens eines vorangegangenen Verfahrensschritts für mindestens ein Fixiermittel eine eigens auf den Einsatz dieses Fixiermittels abgestimmte Fixiermittelposition, die eine Fixierung des Aufsatzes innerhalb der Knochendefektstelle oder an dem gesunden Knochen ermöglicht, vorgegeben, um durch das Fertigungsverfahren einen Aufsatz zu erhalten, an dem mindestens ein Fixiermittel in seiner Fixiermittelposition mittels mindestens einer Verbindung lösbar mit dem Aufsatz verbunden ist.

Nach einer diesbezüglichen vorteilhaften Ausgestaltung des erfindungsgemäßen Verfahrens wird im Fertigungsverfahren an mindestens einer für ein Fixiermittel abgestimmten Fixiermittelposition eine Führung für ein Werkzeug, durch das die Erstellung einer für das Fixiermittel geeigneten Bohrung innerhalb der Knochendefektstelle oder an dem gesunden Knochen ermöglicht wird, angeordnet.

Nach einer zusätzlichen vorteilhaften Ausgestaltung des erfindungsgemäßen Verfahrens entspricht die dem Knochendefekt zugewandte Seite oder die dem Knochendefekt abgewandte Seite des im Fertigungsverfahren gebildeten Aufsatzes zumindest teilweise der Form des regenerierten Knochens, der durch seine Regeneration nach Möglichkeit wieder die Form eines gesunden Knochens aufweist, entspricht, im Soll-Zustand.

Nach einer zusätzlichen vorteilhaften Ausgestaltung des erfindungsgemäßen Verfahrens entsteht der zweite Datensatz durch Berechnung oder wurde zu einer Zeit aufgenommen, zu der der Knochen an der jetzt zu regenerierenden Knochendefektstelle noch ein gesunder Knochen war.

Nach einer zusätzlichen vorteilhaften Ausgestaltung des erfindungsgemäßen Verfahrens entsteht der zweite Datensatz durch Berechnung oder wurde zu einer Zeit aufgenommen, zu der der Knochen an der jetzt zu regenerierenden Knochendefektstelle noch ein gesunder Knochen. Im letzteren Fall ist es möglich, dass ggfls. der Idealzustand (Soll-Zustand) des Knochens dokumentiert wird, so dass bekannt ist, wie ein event. später zu regenerierender Knochen auszusehen hat. Bei Menschen sollte die Aufnahme des Datensatzes des gesunden Knochens bevorzugt im Alter von 18 bis 25 Jahren erfolgen. Selbstverständlich ist es auch denkbar, dass das im ausgewachsenen Zustand mehrere gesunde Knochen oder das gesamte Skelett des Menschen oder des Tieres aufgezeichnet, dokumentiert und/oder gespeichert werden. Denkbar wäre auch, dass bereits zum Zeitpunkt der Aufnahme des gesunden Knochens ein Aufsatz zumindest teilweise angefertigt wird. Bevorzugt erfolgt die Aufnahme des Datensatzes des gesunden Knochens bzw. erfolgen die Aufnahmen der gesunden Knochen, nachdem der gesunde Knochen ausgewachsen ist.

Nach einer diesbezüglichen vorteilhaften Ausgestaltung des erfindungsgemäßen Verfahrens werden der Datensatz des gesunden Knochens bzw. die der Datensätze der gesunden Knochen für seine spätere Verwendung auf einem Speichermedium gespeichert (konserviert).

Nach einer zusätzlichen vorteilhaften Ausgestaltung des erfindungsgemäßen Verfahrens repräsentiert die Aufnahme des ersten Datensatzes die betroffene Knochendefektstelle in ihrer Dreidimensionalität und/oder repräsentiert die Aufnahme des zweiten Datensatzes die Form des noch gesunden Knochens in seiner Dreidimensionalität.

Nach einer zusätzlichen vorteilhaften Ausgestaltung des erfindungsgemäßen Verfahrens erfolgt die Aufnahme des ersten Datensatzes, der den Ist-Zustand repräsentiert, und/oder die Aufnahme des zweiten Datensatzes, der den Soll-Zustand repräsentiert, durch mindestens ein bildgebendes Verfahren.

Nach einer zusätzlichen vorteilhaften Ausgestaltung des erfindungsgemäßen Verfahrens erfolgt die Aufnahme des ersten Datensatzes und/oder die Aufnahme des zweiten Datensatzes mit mindestens einem Verfahren, welches eine dreidimensionale Darstellung eines Knochens ermöglicht. Insbesondere erfolgen die Aufnahme des ersten Datensatzes und/oder die Aufnahme des zweiten Datensatzes mittels Tomografie, Computertomografie, digitaler Volumentomografie, Sonografie odgl..

Nach einer zusätzlichen vorteilhaften Ausgestaltung des erfindungsgemäßen Verfahrens wird im Fertigungsverfahren der Aufsatz mittels 3D-Druck und/oder mittels Fräsung gebildet.

Nach einer zusätzlichen vorteilhaften Ausgestaltung des erfindungsgemäßen Verfahrens werden während der Fertigung des Aufsatzes mindestens eine Befestigungsvorrichtung für mindestens ein zu setzendes Implantat und/oder mindestens eine Sollbruchstelle und/oder mindestens ein Positioniermittel an dem Aufsatz angeordnet. Die Befestigungsvorrichtung kann beispielsweise als Aussparung ausgestaltet sein.

Nach einer diesbezüglichen vorteilhaften Ausgestaltung des erfindungsgemäßen Verfahrens wird mindestens eine Befestigungsvorrichtung (z.B. Aussparung) durch Entfernen eines Teils des Aufsatzes, der vor dem Entfernen mittels mindestens einer Sollbruchstelle mit dem restlichen Teil des Aufsatzes verbunden ist, freigelegt. Der Zeitpunkt der Freilegung der Befestigungsvorrichtung kann dabei vor oder nach der Anordnung der Abdeckvorrichtung an der Knochendefektstelle liegen.

Nach einer zusätzlichen vorteilhaften Ausgestaltung des erfindungsgemäßen Verfahrens dient mindestens ein während der Fertigung des Aufsatzes an dem Aufsatz angeordnetes Positioniermittel zur Positionierung des Aufsatzes in einer Einsetzposition. Bevorzugt ist das Positioniermittel am Rand des Aufsatzes angeordnet. Bevorzugt dient mindestens ein Positioniermittel zur Positionierung des Aufsatzes an einem an die Knochendefektstelle angrenzenden gesunden Knochen, so dass dieses mindestens eine Positioniermittel eine dem gesunden Knochen abgewandte Seite und eine dem gesunden Knochen und mit diesem zumindest teilweise korrespondierende zugewandte Seite aufweist.

Nach einer zusätzlichen vorteilhaften Ausgestaltung des erfindungsgemäßen Verfahrens wird zwischen dem Aufsatz und einem Positioniermittel mindestens eine Sollbruchstelle angeordnet, wodurch bevorzugt ein Positioniermittel, das auf dem gesunden Knochen aufliegt und somit ggfls. störend aufträgt, z.B. nach Fixierung des Aufsatzes oder nach der Regeneration des Knochens an der Knochendefektstelle, von dem eventuell verbleibenden Aufsatz entfernt werden kann, und/oder wird zwischen dem Aufsatz und mindestens einem an dem Aufsatz angeordneten Fixiermittel mindestens eine Sollbruchstelle angeordnet.

Nach einer zusätzlichen vorteilhaften Ausgestaltung des erfindungsgemäßen Verfahrens wird während der Fertigung des Aufsatzes ein Reinigungs- und/oder Sterilisationsprozess durchgeführt.

Nach einer zusätzlichen vorteilhaften Ausgestaltung des erfindungsgemäßen Verfahrens werden der Aufsatz und/oder mindestens ein Fixiermittel und/oder mindestens ein Positioniermittel aus einem formstabilen, insbesondere starren, Material gebildet.

Durch das erfindungsgemäße Verfahren zur Herstellung einer Abdeckvorrichtung für eine Knochendefektstelle kann eine Abdeckvorrichtung geschaffen werden, dessen Aufsatz und/oder Fixiermittel beispielsweise aus einem künstlichen Werkstoff und/oder aus einem Werkstoff autogener, synergener, allogener oder xenogener Herkunft menschliche und/oder tierische Knochen bzw. die menschliche, tierische oder künstliche Matrix eine Form auf, durch die der zwischen dem Knochen und der gewünschten Form des regenerierten Knochens befindlicher Bereich vollständig oder nahezu vollständig ausgefüllt wird. Hierzu wird dem Spender z.B. ein Knochenblock entnommen, der anschließend gegebenenfalls mittels CAD/CAM modelliert wird.

Die erfindungsgemäße Abdeckvorrichtung für eine Knochendefektstelle, die einen Aufsatz, durch den die Knochendefektstelle zumindest teilweise abdeckbar ist, wobei der Aufsatz eine der Knochendefektstelle abgewandte Seite und eine dem Knochendefektstelle zugewandte Seite, oder durch den die Knochendefektstelle zumindest teilweise abdeckbar ist und zusätzlich ein an die Knochendefektstelle angrenzender gesunder Knochen zumindest teilweise abdeckbar ist, wobei der Aufsatz eine dem gesunden Knochen abgewandte Seite und eine der Knochendefektstelle abgewandte Seite und eine dem gesunden Knochen zugewandte Seite und eine der Knochendefektstelle zugewandte Seite aufweist, und ein Fixiermittel zur Fixierung des Aufsatzes innerhalb der Knochendefektstelle und/oder mit mindestens einem Fixiermittel zur Fixierung des Aufsatzes an dem an die Knochendefektstelle angrenzenden gesunden Knochen aufweist, ist an dem Aufsatz mindestens ein zur Fixierung des Aufsatzes innerhalb der Knochendefektstelle oder an dem an die Knochendefektstelle angrenzenden gesunden Knochen geeignetes Fixiermittel mittels mindestens einer zwischen dem Fixiermittel und dem Aufsatz angeordneten Verbindung lösbar angeordnet ist wird, wobei die Lösung mindestens einer Verbindung irreversibel ist und mindestens eine Verbindung eine Sollbruchstelle ist.

Nach einer zusätzlichen vorteilhaften Ausgestaltung der erfindungsgemäßen Abdeckvorrichtung ist an dem Aufsatz für ein Fixiermittel eine eigens auf den Einsatz dieses Fixiermittels abgestimmte Fixiermittelposition vorhanden, die eine Fixierung des Aufsatzes innerhalb der Knochendefektstelle oder an dem gesunden Knochen ermöglicht, oder ist an dem Aufsatz für mindestens ein Fixiermittel mindestens eine eigens auf den Einsatz mindestens eines Fixiermittels abgestimmte Fixiermittelposition vorhanden ist, die eine Fixierung des Aufsatzes innerhalb der Knochendefektstelle oder an dem gesunden Knochen ermöglicht, wobei mindestens ein Fixiermittel in seiner eigens auf den Einsatz dieses Fixiermittels abgestimmten Fixiermittelposition mit dem Aufsatz mittels mindestens einer Verbindung lösbar verbunden ist.

Nach einer zusätzlichen vorteilhaften Ausgestaltung der erfindungsgemäßen Abdeckvorrichtung weist der Aufsatz mindestens eine Sollbruchstelle auf. Die Sollbruchstelle birgt den Vorteil, dass, sofern der Aufsatz nach einer erfolgreichen Knochenregeneration entfernt werden soll, diese Entfernung minimal invasiv erfolgen kann, ohne "alles offen legen zu müssen", da der Aufsatz aufgrund der Sollbruchstelle in mindestens zwei Teile zerkleinert werden kann. Die Entfernung des Aufsatzes (z.B. nach einer Knochenregeneration) ist somit sehr leicht möglich. Des Weiteren kann die Sollbruchstelle dazu dienen, dass nicht oder nicht mehr benötigte Teile des Aufsatzes von dem Rest des Aufsatzes abgetrennt werden.

Nach einer zusätzlichen vorteilhaften Ausgestaltung der erfindungsgemäßen Abdeckvorrichtung überragt mindestens ein Fixiermittel, das bevorzugt in seiner eigens auf den Einsatz dieses Fixiermittels abgestimmten Fixiermittelposition mit dem Aufsatz lösbar verbunden ist, die der Knochendefektstelle abgewandte Seite oder die dem gesunden Knochen abgewandte Seite des Aufsatzes und/oder die der Knochendefektstelle zugewandte Seite oder die dem gesunden Knochen zugewandte Seite des Aufsatzes.

Nach einer zusätzlichen vorteilhaften Ausgestaltung der erfindungsgemäßen Abdeckvorrichtung weist der Aufsatz an mindestens einer für ein Fixiermittel abgestimmten Fixiermittelposition eine Führung für ein Werkzeug auf. Bevorzugt wird durch die Führung für ein Werkzeug die Erstellung einer für das Fixiermittel geeigneten Bohrung innerhalb der Knochendefektstelle oder an dem gesunden Knochen ermöglicht.

Nach einer zusätzlichen vorteilhaften Ausgestaltung der erfindungsgemäßen Abdeckvorrichtung besteht der Aufsatz aus einem formstabilen, insbesondere starren, Material.

Nach einer zusätzlichen vorteilhaften Ausgestaltung der erfindungsgemäßen Abdeckvorrichtung entspricht die dem Knochendefekt zugewandte Seite oder die dem Knochendefekt abgewandte Seite des Aufsatzes zumindest teilweise der Form eines an der Knochendefektstelle regenerierten Knochens, der durch seine Regeneration wieder die Form eines gesunden Knochens aufweist.

Nach einer zusätzlichen vorteilhaften Ausgestaltung der erfindungsgemäßen Abdeckvorrichtung ist der Werkstoff des Aufsatzes organischer und/oder anorganischer Herkunft und/oder ist der Werkstoff eines Fixiermittels organischer und/oder anorganischer Herkunft. Dies kann auch ein autogener, syngener, allogener, xenogener, synthetischer oder alloplastischer Werkstoff sein.

Nach einer zusätzlichen vorteilhaften Ausgestaltung der erfindungsgemäßen Abdeckvorrichtung bestehen der Aufsatz und/oder mindestens ein Fixiermittel mindestens teilweise aus einem biokompatiblen Werkstoff.

Nach einer zusätzlichen vorteilhaften Ausgestaltung der erfindungsgemäßen Abdeckvorrichtung können der Aufsatz und/oder mindestens ein Fixiermittel und/oder mindestens ein Positioniermittel mindestens teilweise aus einem resorbierbaren Werkstoff bestehen. Vorteilhafterweise kann die Resorptionszeit der starren Schale durch deren Resorptionsgradienten gesteuert werden und/oder kann die Resorptionszeit auch weniger als sechs Monate betragen, so dass zeitnah das Implantat eingesetzt werden kann. Bevorzugt werden resorbierbare Metalle oder Legierungen, insbesondere Magnesium oder Magnesiumlegierungen, eingesetzt. Die 3D-Modelle (z.B. der Aufsatz und/oder das Fixiermittel) werden bevorzugt im Laserschmelz-Verfahren (Laser-melting-Verfahren) unter Vakuum aufgebaut, wobei bevorzugt ein 3D-Drucker zum Einsatz kommt.

Nach einer zusätzlichen vorteilhaften Ausgestaltung der erfindungsgemäßen Abdeckvorrichtung bestehen der Aufsatz und/oder mindestens ein Fixiermittel und/oder mindestens ein Positioniermittel mindestens teilweise aus einem biodegradierbaren Werkstoff.

Nach einer zusätzlichen vorteilhaften Ausgestaltung der erfindungsgemäßen Abdeckvorrichtung bestehen der Aufsatz und/oder mindestens ein Fixiermittel und/oder mindestens ein Positioniermittel mindestens teilweise aus einem Polymer oder einer polymeren Verbindung.

Nach einer zusätzlichen vorteilhaften Ausgestaltung der erfindungsgemäßen Abdeckvorrichtung bestehen der Aufsatz und/oder mindestens ein Fixiermittel und/oder mindestens ein Positioniermittel mindestens teilweise aus einem biokompatiblen Werkstoff.

Nach einer zusätzlichen vorteilhaften Ausgestaltung der erfindungsgemäßen Abdeckvorrichtung bestehen der Aufsatz und/oder mindestens ein Fixiermittel und/oder mindestens ein Positioniermittel mindestens teilweise aus einem resorbierbaren oder nicht resorbierbaren Polymer (z.B. Polylactid (PLA), Polycaprolactone (PCL), bioresorbierbare Legierungen (z.B. auf Magnesium-Basis)).

Nach einer zusätzlichen vorteilhaften Ausgestaltung der erfindungsgemäßen Abdeckvorrichtung weisen der Aufsatz und/oder ein Positioniermittel eine Wandstärke von mindestens 0,1 mm auf, bevorzugt 0,2 - 0,3 mm insbesondere bei Titan und 0,5 - 1 mm insbesondere bei Polycaprolactonen, auf, jedoch zumindest so viel, so dass sich eine Formstabilität des Aufsatzes bzw. eines Positioniermittels ergibt.

Nach einer zusätzlichen vorteilhaften Ausgestaltung der erfindungsgemäßen Abdeckvorrichtung weist der Aufsatz und/oder mindestens ein Positioniermittel eine konstante oder eine variierende Wandstärke auf.

Nach einer zusätzlichen vorteilhaften Ausgestaltung der erfindungsgemäßen Abdeckvorrichtung ist mindestens ein Fixiermittel ein Pin, eine Schraube, ein Nagel und/oder ein Knochenkleber. Um den gesunden Knochen zu schonen, wird das bzw. werden die Fixiermittel bevorzugt im Bereich der Knochendefektstelle angeordnet.

Nach einer zusätzlichen vorteilhaften Ausgestaltung der erfindungsgemäßen Abdeckvorrichtung weist der Aufsatz mindestens eine Fräsung (Bohrung für das Fixiermittel) auf.

Nach einer zusätzlichen vorteilhaften Ausgestaltung der erfindungsgemäßen Abdeckvorrichtung korrespondiert die Fräsung mit dem Fixiermittel.

Nach einer zusätzlichen vorteilhaften Ausgestaltung der erfindungsgemäßen Abdeckvorrichtung weist die dem Knochendefekt zugewandte Wandung eine Oberflächenkonditionierung auf.

Nach einer zusätzlichen vorteilhaften Ausgestaltung der erfindungsgemäßen Abdeckvorrichtung kann die Oberfläche eine Mikrostrukturierung, Poren, Osteoblastenlockstoffe, Mittel zur Förderung des Knochenwachstums und/oder BMP-haltiges Knochenersatzmittel aufweisen.

Nach einer zusätzlichen vorteilhaften Ausgestaltung der erfindungsgemäßen Abdeckvorrichtung weist der Aufsatz mindestens eine Öffnung auf. Dies bedeutet, dass der Aufsatz keine geschlossene Wandung aufweisen muss. Durch eine Vielzahl von Öffnungen kann der Aufsatz zumindest stellenweise eine Netzstruktur aufweisen, wobei die dem Knochendefekt abgewandte Seite des Aufsatzes der Netzstruktur oder die dem Knochendefekt zugewandte Seite des Aufsatzes der Netzstruktur der Form des regenerierten Knochens entspricht.

Nach einer zusätzlichen vorteilhaften Ausgestaltung der erfindungsgemäßen Abdeckvorrichtung weist der Aufsatz mindestens eine Befestigungsvorrichtung (z.B. eine Aussparung) für mindestens ein zu setzendes Implantat auf.

Nach einer zusätzlichen vorteilhaften Ausgestaltung der erfindungsgemäßen Abdeckvorrichtung ist mindestens eine Befestigungsvorrichtung (z.B. eine Aussparung) durch einen Teil des Aufsatzes, der mittels mindestens einer Sollbruchstelle mit dem restlichen Teil des Aufsatzes verbunden ist, zumindest teilweise abgedeckt.

Nach einer zusätzlichen vorteilhaften Ausgestaltung der erfindungsgemäßen Abdeckvorrichtung ist zur Positionierung des Aufsatzes an einem an die Knochendefektstelle angrenzenden gesunden Knochen an dem Aufsatz mindestens ein Positioniermittel angeordnet, das eine dem gesunden Knochen abgewandte Seite und eine dem gesunden Knochen und mit diesem zumindest teilweise korrespondierende zugewandte Seite aufweist.

Nach einer zusätzlichen vorteilhaften Ausgestaltung der erfindungsgemäßen Abdeckvorrichtung ist zwischen dem Aufsatz und mindestens einem Positioniermittel mindestens eine Sollbruchstelle angeordnet.

Nach einer zusätzlichen vorteilhaften Ausgestaltung der erfindungsgemäßen Abdeckvorrichtung wird die Abdeckvorrichtung gemäß dem Verfahren zur Herstellung einer Abdeckvorrichtung für eine Knochendefektstelle nach einem der Ansprüche 1 bis 14 hergestellt.

Mit der erfindungsgemäßen Abdeckvorrichtung kann ein Verfahren zur zumindest teilweisen Abdeckung einer Knochendefektstelle mittels einer hierfür in eine Einsetzposition verbrachten Abdeckvorrichtung für eine Knochendefektstelle, die einen Aufsatzes und mindestens ein Fixiermittel für den Aufsatz aufweist, durchgeführt werden
- wobei der Aufsatz dazu geeignet ist, die Knochendefektstelle zumindest teilweise abzudecken, wobei der Aufsatz eine der Knochendefektstelle abgewandte Seite und eine der Knochendefektstelle zugewandte Seite aufweist und der Aufsatz mit mindestens einem Fixiermittel innerhalb der Knochendefektstelle fixierbar ist,
   oder
- wobei der Aufsatz dazu geeignet ist, die Knochendefektstelle zumindest teilweise abzudecken und zusätzlich einen an die Knochendefektstelle angrenzenden gesunden Knochen zumindest teilweise abzudecken, wobei der Aufsatz eine dem gesunden Knochen abgewandte Seite und eine der Knochendefektstelle abgewandte Seite und eine dem gesunden Knochen zugewandte Seite und eine der Knochendefektstelle zugewandte Seite aufweist und der Aufsatz mit mindestens einem Fixiermittel innerhalb der Knochendefektstelle und/oder mit mindestens einem Fixiermittel an dem gesunden Knochen fixierbar ist,
ist an dem Aufsatz mindestens ein zur Fixierung des Aufsatzes geeignetes Fixiermittel mittels mindestens einer Verbindung lösbar angeordnet, wobei eine Verbindung zwischen dem Fixiermittel und dem Aufsatz gelöst wird, wobei die Lösung mindestens einer Verbindung irreversibel ist und mindestens eine Verbindung eine Sollbruchstelle ist, wodurch der Aufsatz mittels dieses Fixiermittels innerhalb der Knochendefektstelle oder an dem gesunden Knochen fixierbar ist.

Nach einer vorteilhaften Ausgestaltung des Verfahrens ist an dem Aufsatz für mindestens ein Fixiermittel mindestens eine eigens auf den Einsatz mindestens eines Fixiermittels abgestimmte Fixiermittelposition, die eine Fixierung des Aufsatzes innerhalb der Knochendefektstelle oder an dem gesunden Knochen ermöglicht, vorgegeben, wobei mindestens ein Fixiermittel in seiner eigens auf den Einsatz dieses Fixiermittels abgestimmten Fixiermittelposition, die eine Fixierung des Aufsatzes innerhalb der Knochendefektstelle oder an dem gesunden Knochen ermöglicht, mit dem Aufsatz lösbar verbunden ist, so dass zur Fixierung des Aufsatzes innerhalb der Knochendefektstelle oder an dem gesunden Knochen mittels dieses lösbar mit dem Aufsatz verbundenen Fixiermittels die Verbindung zwischen dem Fixiermittel und dem Aufsatz gelöst wird, wodurch der Aufsatz mittels dieses Fixiermittels innerhalb der Knochendefektstelle oder an dem gesunden Knochen fixierbar ist.

Nach einer zusätzlichen vorteilhaften Ausgestaltung des Verfahrens ist das Fixiermittel mittels mindestens einer Sollbruchstelle mit dem Aufsatz lösbar verbunden, so dass die Lösung der Verbindung zwischen dem Fixiermittel und dem Aufsatz durch Brechen der mindestens einen Sollbruchstelle erfolgt.

Nach einer zusätzlichen vorteilhaften Ausgestaltung des Verfahrens ist nach dem Lösen des Fixiermittels von dem Aufsatz der Aufsatz sofort mittels diesem Fixiermittels fixierbar oder wird nach dem Lösen des Fixiermittels von dem Aufsatz das Fixiermittels von der Fixiermittelposition entfernt, um vor einer dadurch möglichen Fixierung des Aufsatzes mittels diesem wieder in die Fixiermittelposition verbrachten Fixiermittels mindestens einen Verfahrensschritt durchzuführen.

Nach einer zusätzlichen vorteilhaften Ausgestaltung des Verfahrens ist an dem Aufsatz mindestens ein Positioniermittel angeordnet, wobei mindestens ein Positioniermittel dazu eingesetzt wird, um die Abdeckvorrichtung in die Einsetzposition zu verbringen. Bevorzugt wird mindestens ein Positioniermittel von dem Aufsatz entfernt, nachdem die Abdeckvorrichtung in die Einsetzposition verbracht wurde. Durch das mindestens eine Positioniermittel ist bevorzugt eine Positionierung des Aufsatzes an einem an die Knochendefektstelle angrenzenden gesunden Knochen möglich, da das Positioniermittel eine dem gesunden Knochen abgewandte Seite und eine dem gesunden Knochen und mit diesem zumindest teilweise korrespondierende zugewandte Seite aufweist. Der Aufsatz ist dabei bevorzugt ausschließlich im Bereich der Knochendefektstelle, die von dem Aufsatz vollständig oder zumindest teilweise abgedeckt wird, angeordnet und/oder fixiert, so dass er den an die Knochendefektstelle angrenzenden gesunden Knochen, an dem aufgrund seiner Gesundheit sowieso keine Regeneration des Knochens stattfindet, nicht in Mitleidenschaft zieht. Statt der nur teilweisen Abdeckung der Knochendefektstelle durch den Aufsatz ist der Aufsatz somit bevorzugt passgenau auf die Knochendefektstelle abgestimmt und endet bevorzugt bündig am gesunden Knochen. Hierdurch ist die Knochendefektstelle vollständig durch den bevorzugt nicht über die Knochendefektstelle hinausragenden Aufsatz abgedeckt.

Nach einer zusätzlichen vorteilhaften Ausgestaltung des Verfahrens wird als Abdeckvorrichtung eine Abdeckvorrichtung eingesetzt, die gemäß dem Verfahren zur Herstellung einer Abdeckvorrichtung für eine Knochendefektstelle nach einem der Ansprüche 1 bis 14 hergestellt wurde und/oder wird als Abdeckvorrichtung eine Abdeckvorrichtung eingesetzt, die eine Abdeckvorrichtung nach einem der Ansprüche 15 bis 27 ist.

Weitere Vorteile und vorteilhafte Ausgestaltungen der Erfindung sind der nachfolgenden Beschreibung, der Zeichnung und den Ansprüchen entnehmbar.

### Zeichnung

Ausführungsbeispiele des Gegenstandes der Erfindung sind in der Zeichnung dargestellt und werden im Folgenden näher erläutert. Es zeigen:
- Fig. 1: eine perspektivische Darstellung einer erfindungsgemäßen Abdeckvorrichtung für eine Knochendefektstelle,
- Fig. 2: eine andere perspektivische Darstellung der erfindungsgemäßen Abdeckvorrichtung für eine Knochendefektstelle, gemäß Fig. 1,
- Fig. 3: eine andere perspektivische Darstellung der erfindungsgemäßen Abdeckvorrichtung für eine Knochendefektstelle, gemäß Fig. 1,
- Fig. 4: eine Aufsicht auf die erfindungsgemäße Abdeckvorrichtung für eine Knochendefektstelle, gemäß Fig. 1, und
- Fig. 5: eine Detailansicht der erfindungsgemäßen Abdeckvorrichtung für eine Knochendefektstelle, gemäß Fig. 1.

### Beschreibung der Ausführungsbeispiele

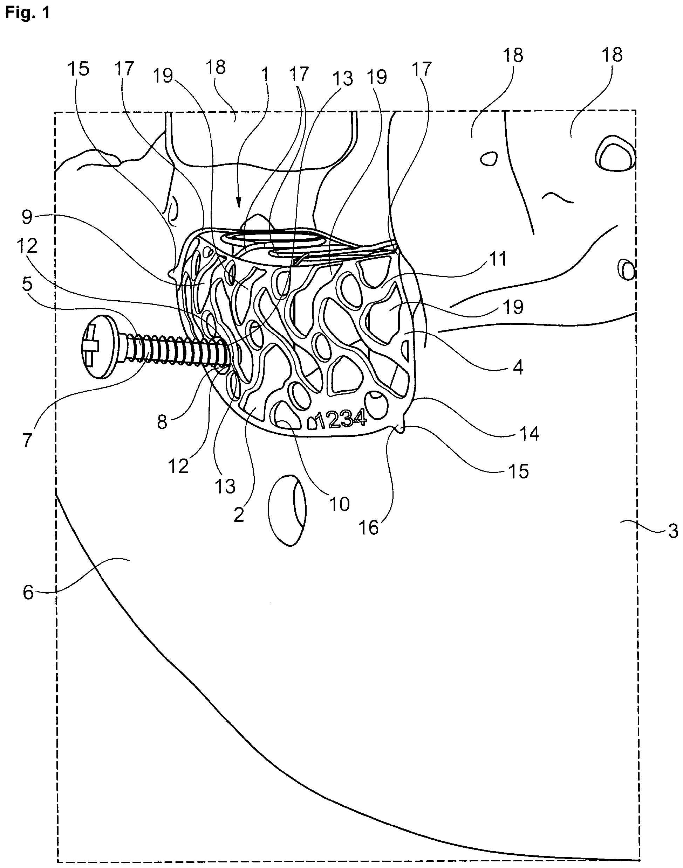

Fig. 1 zeigt eine perspektivische Darstellung einer erfindungsgemäßen Abdeckvorrichtung 1 für eine Knochendefektstelle 2 (Knochendefekt) eines Knochens, insbesondere eines Kieferknochens 3. Die erfindungsgemäße Abdeckvorrichtung 1, die zur Abdeckung und/oder Rekonstruktion einer Knochendefektstelle 2 dient, besteht aus einem Aufsatz 4, der einschichtig ist, und einem Fixiermittel 5, das in Fig. 1 als Schraube dargestellt ist und das, um den an die Knochendefektstelle 2 angrenzenden gesunden Knochen nicht zu verletzen, in der Knochendefektstelle 2 anordbar ist. Selbstverständlich ist es auch denkbar, das mehrere Fixiermittel 5 zur Fixierung des Aufsatzes 4 eingesetzt werden, wobei diese bevorzugt ebenfalls in der Knochendefektstelle 2 angeordnet wären. Der Aufsatz 4 ist aus einem formstabilen, insbesondere starren, Material, so dass er selbsttragend ist und keine zusätzliche Abstützung notwendig ist. Zur Fixierung des Aufsatzes 4 (Formschale, starre Schale) wird das Fixiermittel 5 durch eine Bohrung 6 in den Kieferknochen 3 eingeschraubt. Bevorzugt wird hierfür im Kieferknochen 3 eine nicht dargestellte Bohrung erzeugt. Denkbar ist auch, dass anstelle der Schraube 7 ein Pin oder ein Pin mit mindestens einem Widerhaken als Fixiermittel 5 eingesetzt wird. In diesem Fall erfolgt die anschließende Fixierung des Aufsatzes 4 bevorzugt über Ultraschall-Schweißen. Beim Ultraschall-Schweißen erzeugt bevorzugt ein Ultraschallgenerator eine genau definierte Frequenz, welche über eine Sonotrode gebündelt wird. Nach dem Aufsetzen des resorbierbaren Fixiermittels 5 (Pin) auf ein im Knochen vorgebohrtes Bohrloch (Bohrung) sorgt eine erzeugte Schwingung für eine Verflüssigung der Pinoberflächen an dessen Rändern, wodurch ein Eingleiten des Pins in das Bohrloch bewirkt wird. Durch die Änderung des Aggregatzustandes dringt der Pin auch in die knöchernen Hohlräume vor, die von einer gewöhnlichen Knochenschraube (Schraube 7) unerreichbar sind, so dass eine hohe initiale Festigkeit erzielt wird. Zudem verbindet sich der Pinkopf mit dem Aufsatz 4 und sorgt mit einem Verblockungsmechanismus für ein stabiles dreidimensionales Konstrukt. Beim Ultraschall-Schweißen wird somit das Fixiermittel 5 aufgeweicht, so dass es sich mit dem Kieferknochen 3 und dem Aufsatz 4 verbindet.

Durch den fixierten Aufsatz 4 entsteht ein abgedichteter Innenraum 9 zwischen dem Kieferknochen 3 und dem Aufsatz 4, der durch die Regeneration des Knochens und/oder durch Einbringung eines Materials organischer und/oder anorganischer Herkunft, das auch ein autogener, syngener, allogener, xenogener, synthetischer und/oder alloplastischer Werkstoff sein kann, ausgefüllt wird, so dass der regenerierte Knochen oder das eingebrachte Material der Form der Knochendefektstelle 2 zugewandten Seite 10 des einschichtigen Aufsatzes 4 entspricht. Denkbar wäre auch, dass die der Knochendefektstelle 2 abgewandte Seite 11 des einschichtigen Aufsatzes 4 dem der Form der Knochendefektstelle 2 entspricht. Um den Regenerationsprozess des Kieferknochens 3 zu beschleunigen, kann die dem Knochendefekt zugewandte Seite 10 des Aufsatzes 4 eine Oberflächenkonditionierung (z.B. eine Mikrostrukturierung, Poren, Osteoblastenlockstoffe, Mittel zur Förderung des Knochenwachstums und/oder BMP-haltiges Knochenersatzmittel) aufweisen.

Um die Handhabung des Fixiermittels 5 zu erleichtern und/oder um Sicherzustellen, dass das Fixiermittel 5 an seiner geplanten Fixiermittelposition an dem Aufsatz angeordnet wird, ist das Fixiermittel 5 lösbar durch mindestens eine Verbindung 12 mit dem Aufsatz 4 verbunden. Dies bedeutet, dass das Fixiermittel 5 schon vor dem Einsatz des Aufsatzes 4 in die Knochendefektstelle mit dem Aufsatz 4 verbunden ist. Fig. 1 zeigt die Abdeckvorrichtung 1 nach ihrem Einsatz in die Knochendefektstelle 2, wobei die Abdeckvorrichtung 1 noch nicht mit dem Fixiermittel 5 an der Knochendefektstelle 2 fixiert ist. Bei der Verwendung des Fixiermittels 5 wird die Verbindung 12 beispielsweise durch Druck auf das Fixiermittel 5 und/oder einem Verdrehen des Fixiermittels 5 irreversibel (unumkehrbar) getrennt, so dass das Fixiermittel 5 nicht mehr mittels der Verbindung 12 mit dem Aufsatz 4 verbunden werden kann. Bevorzugt ist das Fixiermittel 5 mittels mindestens einer Verbindung 12, die z.B. durch mindestens einen Steg, der als Sollbruchstelle 13 fungiert, realisiert sein kann, mit dem Aufsatz 4 verbunden. Nach dem Lösen der Verbindung 12, also z.B. durch Brechen des Steges bzw. der Sollbruchstelle 13, kann das Fixiermittel 5 bestimmungsgemäß verwendet werden. Denkbar ist auch, dass mehrere Fixiermittel 5 eingesetzt werden, wobei mindestens ein Fixiermittel 5 mittels mindestens einer Verbindung 12 lösbar mit dem Aufsatz verbunden ist, wobei die Lösung der Verbindung 12 irreversibel ist, so dass mindestens ein Fixiermittel 5 nicht mehr mittels der Verbindung 12 mit dem Aufsatz 4 verbunden werden kann.

Am Rand 14 des Aufsatzes 4 sind Positioniermittel 15, die eine einem gesunden Knochen 6 zugewandte Seite und eine dem gesunden Knochen 6 abgewandte Seite 16 aufweisen. Bei der ordnungsgemäßen Anordnung des Aufsatzes 4 an der Knochendefektstelle 2, die durch die Positioniermittel 15 unterstützt wird, berühren die einem gesunden Knochen zugewandten Seite zumindest teilweise den gesunden Knochen 6, wodurch durch die Positioniermittel 15 ein einwandfreier Sitz des Aufsatzes 4 ggfls. selbst ohne Anordnung mindestens eines Fixiermittels 5 oder zumindest bis mindestens ein Fixiermittel 5 in der Knochendefektstelle 2 angeordnet ist, gewährleistet wird. Um den Aufsatz 4 nach der Knochenregeneration minimal invasiv leicht entfernen zu können, weist dieser Sollbruchstellen 17 auf, wodurch er nach deren Durchtrennung für seine Entnahme z.B. in zwei Teile geteilt werden kann.

Fig. 1 zeigt den an der Knochendefektstelle 2 des ausschnittsweise dargestellten Kieferknochens 3, der Zähne 18 aufweist, angeordneten Aufsatz 4. Hierdurch wird ersichtlich, dass der Aufsatz 4 bevorzugt nur im Bereich der Knochendefektstelle 2 des Kieferknochens 3 angeordnet ist, so dass er einen gesunden Knochen 6 weder überspannt noch berührt. Somit haben nur die an dem Aufsatz 4 angeordneten Positioniermittel 15 Kontakt mit dem gesunden Knochen 19. Denkbar ist, dass zwischen dem Positioniermittel 15 und dem Aufsatz 4 mindestens eine nicht dargestellte Sollbruchstelle angeordnet ist, wodurch das Positioniermittel 15 nach der Fixierung des Aufsatzes 4 in der Knochendefektstelle 2 von dem Aufsatz 4 abgetrennt werden kann.

In Fig. 1 ist ein Aufsatz 4 dargestellt, dessen dem Knochendefekt zugewandte Seite 10 der Form des regenerierten Knochens entspricht. Denkbar ist auch, dass die Positioniermittel 15 derart an dem Aufsatz 4 angeordnet werden, dass die dem Knochendefekt abgewandte Seite 11 des Aufsatzes 4 der Form des regenerierten Knochens entspricht. Bewerkstelligt werden könnte dies z.B. durch Anordnung der Positioniermittel 15 an der dem Knochendefekt abgewandten Seite 11 des Aufsatzes 4.

Schematisch dargestellt sind die Bereiche 19, die die Position der zukünftig einzusetzenden Implantate darstellen.

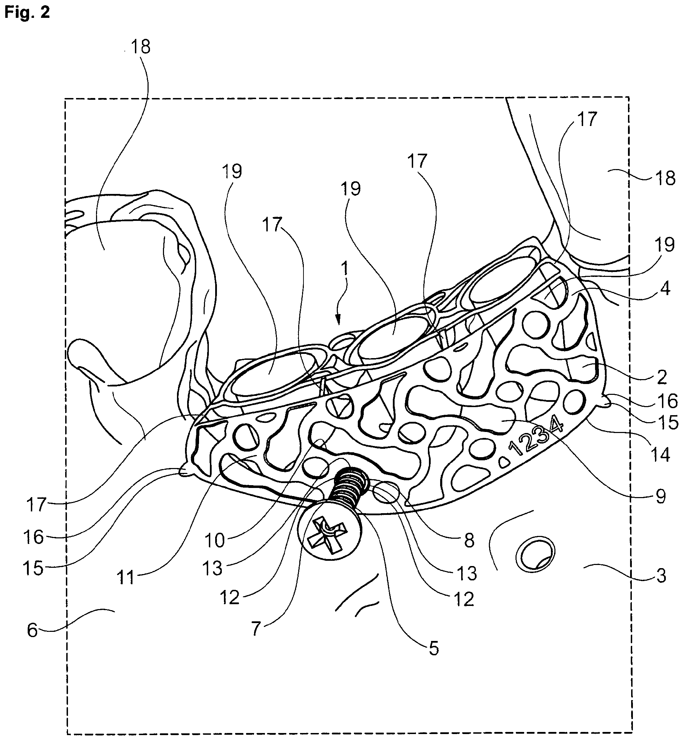

Fig. 2 zeigt eine andere perspektivische Darstellung der erfindungsgemäßen Abdeckvorrichtung 1 für eine Knochendefektstelle 2, gemäß Fig. 1.

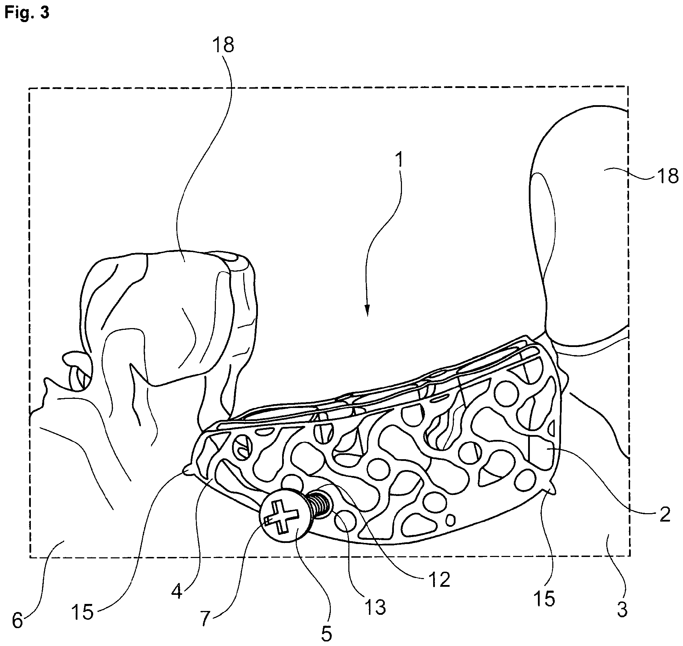

Fig. 3 zeigt eine andere perspektivische Darstellung der erfindungsgemäßen Abdeckvorrichtung 1 für eine Knochendefektstelle 2, gemäß Fig. 1.

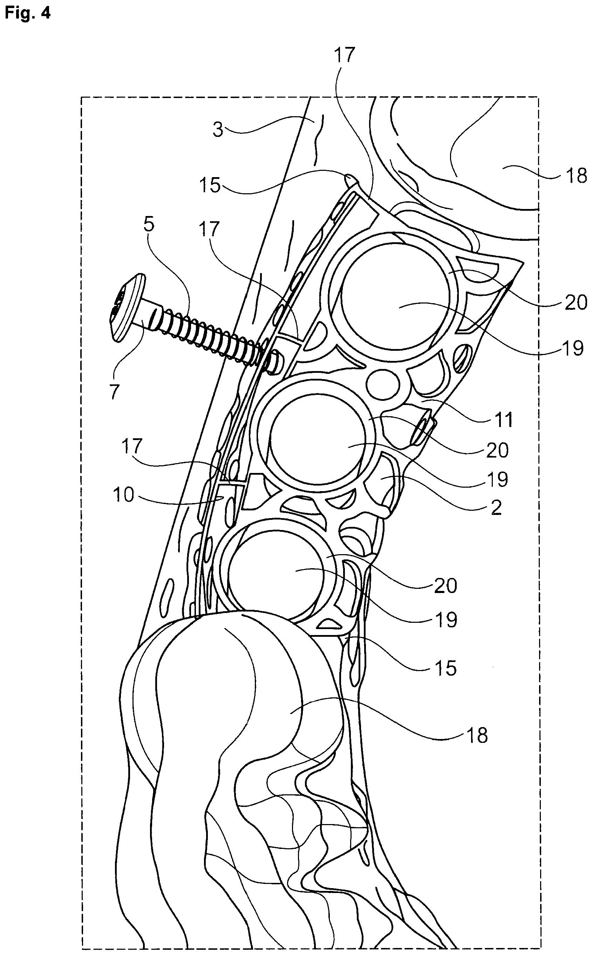

Fig. 4 zeigt eine Aufsicht auf die erfindungsgemäße Abdeckvorrichtung 1 für eine Knochendefektstelle 2, gemäß Fig. 1. Zum zukünftigen Einsetzen der Implantate weist der Aufsatz 4 Öffnungen 20 auf.

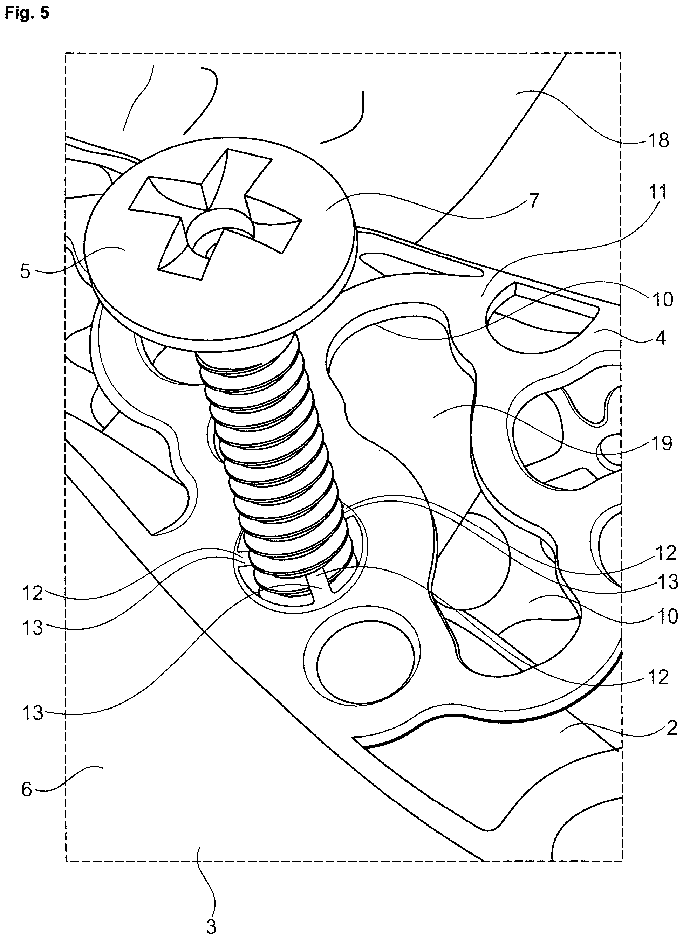

Fig. 5 zeigt eine Detailansicht der erfindungsgemäßen Abdeckvorrichtung 1 für eine Knochendefektstelle 2, gemäß Fig. 1.

### Bezugszahlenliste

- 1: Abdeckvorrichtung
- 2: Knochendefektstelle
- 3: Kieferknochen
- 4: Aufsatz
- 5: Fixiermittel
- 6: Gesunder Knochen
- 7: Schraube
- 8: Bohrung
- 9: Innenraum
- 10: Seite
- 11: Seite
- 12: Verbindung
- 13: Sollbruchstelle
- 14: Rand
- 15: Positioniermittel
- 16: Seite
- 17: Sollbruchstelle
- 18: Zahn
- 19: Bereich
- 20: Öffnungen

## Patentansprüche

1. Verfahren zur Herstellung einer Abdeckvorrichtung für eine Knochendefektstelle (2), die einen Aufsatz (4) und mindestens ein Fixiermittel (5) für den Aufsatz (4) aufweist, bestehend aus folgenden Verfahrensschritten:
- Aufnahme eines ersten Datensatzes, der die betroffene Knochendefektstelle (2) im Ist-Zustand repräsentiert,
- Vergleich des ersten Datensatzes mit einem zweiten Datensatz, der den Soll-Zustand eines an der Knochendefektstelle (2) regenerierten Knochens repräsentiert,
- Verwendung des ersten Datensatzes und des zweiten Datensatzes zur Planung des Aufsatzes (4),
- der dazu geeignet ist, die Knochendefektstelle (2) zumindest teilweise abzudecken, wobei der Aufsatz (4) eine der Knochendefektstelle (2) abgewandte Seite (11) und eine der Knochendefektstelle (2) zugewandte Seite (10) aufweist und der Aufsatz (4) mit mindestens einem Fixiermittel (5) innerhalb der Knochendefektstelle (2) fixierbar ist,
oder
- der dazu geeignet ist, die Knochendefektstelle (2) zumindest teilweise abzudecken und zusätzlich einen an die Knochendefektstelle (2) angrenzenden gesunden Knochen (6) zumindest teilweise abzudecken, wobei der Aufsatz (4) eine dem gesunden Knochen (6) abgewandte Seite und eine der Knochendefektstelle (2) abgewandte Seite (11) und eine dem gesunden Knochen (6) zugewandte Seite und eine der Knochendefektstelle (2) zugewandte Seite (10) aufweist und der Aufsatz (4) mit mindestens einem Fixiermittel (5) innerhalb der Knochendefektstelle (2) und/oder mit mindestens einem Fixiermittel (5) an dem gesunden Knochen (6) fixierbar ist,
- Umsetzung der Planung des Aufsatzes (4) in einen Planungsdatensatz und
- Zuführung des Planungsdatensatzes an ein Fertigungsverfahren, insbesondere an ein computergesteuertes Fertigungsverfahren, wobei während und/oder nach der Fertigung des Aufsatzes (4) mindestens ein zur Fixierung des Aufsatzes (4) innerhalb der Knochendefektstelle (2) oder an dem an die Knochendefektstelle (2) angrenzenden gesunden Knochen (6) geeignetes Fixiermittel (5) mittels mindestens einer zwischen dem Fixiermittel (5) und dem Aufsatz (4) angeordneten Verbindung (12) lösbar mit dem Aufsatz (4) verbunden wird, wobei die Lösung mindestens einer Verbindung (12) irreversibel ist, **dadurch gekennzeichnet, dass** mindestens eine Verbindung (12) eine Sollbruchstelle (13) ist.

2. Verfahren, nach Anspruch 1, **dadurch gekennzeichnet, dass** während mindestens eines vorangegangenen Verfahrensschritts für mindestens ein Fixiermittel (5) eine eigens auf den Einsatz dieses Fixiermittels (5) abgestimmte Fixiermittelposition, die eine Fixierung des Aufsatzes (4) innerhalb der Knochendefektstelle (2) oder an dem gesunden Knochen (6) ermöglicht, vorgegeben ist, um durch das Fertigungsverfahren einen Aufsatz (4) zu erhalten, an dem mindestens ein Fixiermittel (5) in seiner Fixiermittelposition lösbar mit dem Aufsatz (4) verbunden ist.

3. Verfahren, nach Anspruch 2, **dadurch gekennzeichnet, dass** im Fertigungsverfahren an mindestens einer für ein Fixiermittel (5) abgestimmten Fixiermittelposition eine Führung für ein Werkzeug, durch das die Erstellung einer für das Fixiermittel (5) geeigneten Bohrung innerhalb der Knochendefektstelle (2) oder an dem gesunden Knochen (6) ermöglicht wird, angeordnet wird.

4. Verfahren, nach einem der vorhergehenden Ansprüche, **dadurch gekennzeichnet, dass** die dem Knochendefekt zugewandte Seite (10) oder die dem Knochendefekt abgewandte Seite (11) des im Fertigungsverfahren gebildeten Aufsatzes (4) zumindest teilweise der Form des regenerierten Knochens im Soll-Zustand entspricht.

5. Verfahren, nach einem der vorhergehenden Ansprüche, **dadurch gekennzeichnet, dass** der zweite Datensatz durch Berechnung entsteht oder zu einer Zeit aufgenommen wurde, zu der der Knochen an der jetzt zu regenerierenden Knochendefektstelle (2) noch ein gesunder Knochen (6) war.

6. Verfahren, nach einem der vorhergehenden Ansprüche, **dadurch gekennzeichnet, dass** die Aufnahme des ersten Datensatzes die betroffene Knochendefektstelle (2) in ihrer Dreidimensionalität repräsentiert und/oder die Aufnahme des zweiten Datensatzes die Form des noch gesunden Knochens (6) in seiner Dreidimensionalität repräsentiert.

7. Verfahren, nach einem der vorhergehenden Ansprüche, **dadurch gekennzeichnet, dass** die Aufnahme des ersten Datensatzes und/oder die Aufnahme des zweiten Datensatzes durch mindestens ein bildgebendes Verfahren erfolgt.

8. Verfahren, nach einem der vorhergehenden Ansprüche, **dadurch gekennzeichnet, dass** die Aufnahme des ersten Datensatzes und/oder die Aufnahme des zweiten Datensatzes mit mindestens einem Verfahren, welches eine dreidimensionale Darstellung eines Knochens ermöglicht, erfolgt.

9. Verfahren, nach einem der vorhergehenden Ansprüche, **dadurch gekennzeichnet, dass** im Fertigungsverfahren der Aufsatz (4) mittels 3D-Druck und/oder mittels Fräsung gebildet wird.

10. Verfahren, nach einem der vorhergehenden Ansprüche, **dadurch gekennzeichnet, dass** während der Fertigung des Aufsatzes (4) mindestens eine Befestigungsvorrichtung für mindestens ein zu setzendes Implantat und/oder mindestens eine Sollbruchstelle (13, 17) und/oder mindestens ein Positioniermittel (15) an dem Aufsatz (4) angeordnet werden.

11. Verfahren, nach Anspruch 10, **dadurch gekennzeichnet, dass** mindestens eine Befestigungsvorrichtung durch Entfernen eines Teils des Aufsatzes (4), der vor dem Entfernen mittels mindestens einer Sollbruchstelle (17) mit dem restlichen Teil des Aufsatzes (4) verbunden ist, freigelegt wird.

12. Verfahren, nach Anspruch 10 oder Anspruch 11, **dadurch gekennzeichnet, dass** mindestens ein während der Fertigung des Aufsatzes (4) an dem Aufsatz (4) angeordnetes Positioniermittel (15) zur Positionierung des Aufsatzes (4) in einer Einsetzposition dient.

13. Verfahren, nach einem der Ansprüche 10 bis 12, **dadurch gekennzeichnet, dass** zwischen dem Aufsatz (4) und mindestens einem Positioniermittel (15) mindestens eine Sollbruchstelle (17) angeordnet wird und/oder dass zwischen dem Aufsatz (4) und mindestens einem an dem Aufsatz (4) angeordneten Fixiermittel (5) mindestens eine Sollbruchstelle (13) angeordnet wird.

14. Verfahren, nach einem der vorhergehenden Ansprüche, **dadurch gekennzeichnet, dass** der Aufsatz (4) und/oder mindestens ein Fixiermittel (5) und/oder mindestens ein Positioniermittel (15) aus einem formstabilen Material gebildet werden.

15. Abdeckvorrichtung (1) für eine Knochendefektstelle (2),
- mit einem Aufsatz (4),
- durch den die Knochendefektstelle (2) zumindest teilweise abdeckbar ist, wobei der Aufsatz (4) eine der Knochendefektstelle (2) abgewandte Seite (11) und eine dem Knochendefektstelle (2) zugewandte Seite (10),
oder
- durch den die Knochendefektstelle (2) zumindest teilweise abdeckbar ist und zusätzlich ein an die Knochendefektstelle (2) angrenzender gesunder Knochen (6) zumindest teilweise abdeckbar ist, wobei der Aufsatz (4) eine dem gesunden Knochen (6) abgewandte Seite und eine der Knochendefektstelle (2) abgewandte Seite (11) und eine dem gesunden Knochen (6) zugewandte Seite und eine der Knochendefektstelle (2) zugewandte Seite (10),
aufweist,
- mit mindestens einem Fixiermittel (5) zur Fixierung des Aufsatzes (4) innerhalb der Knochendefektstelle (2) und/oder mit mindestens einem Fixiermittel (5) zur Fixierung des Aufsatzes (4) an dem an die Knochendefektstelle (2) angrenzenden gesunden Knochen (6),
**dadurch gekennzeichnet,**
**dass** an dem Aufsatz (4) mindestens ein zur Fixierung des Aufsatzes (4) innerhalb der Knochendefektstelle (2) oder an dem an die Knochendefektstelle (2) angrenzenden gesunden Knochen (6) geeignetes Fixiermittel (5) mittels mindestens einer zwischen dem Fixiermittel (5) und dem Aufsatz (4) angeordneten Verbindung (12) lösbar angeordnet ist wird, wobei die Lösung mindestens einer Verbindung (12) irreversibel ist, **dadurch gekennzeichnet, dass** mindestens eine Verbindung (12) eine Sollbruchstelle (13) ist.

16. Abdeckvorrichtung (1), nach Anspruch 15, **dadurch gekennzeichnet, dass** an dem Aufsatz (4) für ein Fixiermittel (5) eine eigens auf den Einsatz dieses Fixiermittels (5) abgestimmte Fixiermittelposition vorhanden ist, die eine Fixierung des Aufsatzes (4) innerhalb der Knochendefektstelle (2) oder an dem gesunden Knochen (6) ermöglicht, oder an dem Aufsatz (4) für mindestens ein Fixiermittel (5) mindestens eine eigens auf den Einsatz mindestens eines Fixiermittels (5) abgestimmte Fixiermittelposition vorhanden ist, die eine Fixierung des Aufsatzes (4) innerhalb der Knochendefektstelle (2) oder an dem gesunden Knochen (6) ermöglicht, wobei mindestens ein Fixiermittel (5) in seiner eigens auf den Einsatz dieses Fixiermittels (5) abgestimmten Fixiermittelposition mit dem Aufsatz (4) lösbar verbunden ist.

17. Abdeckvorrichtung (1), nach Anspruch 15 oder Anspruch 16, **dadurch gekennzeichnet, dass** der Aufsatz (4) mindestens eine Sollbruchstelle (17) aufweist.

18. Abdeckvorrichtung (1), nach einem der Ansprüche 15 bis 17, **dadurch gekennzeichnet, dass** mindestens ein Fixiermittel (5)
- die der Knochendefektstelle (2) abgewandte Seite (11) oder die dem gesunden Knochen (6) abgewandte Seite des Aufsatzes (4)
und/oder
- die der Knochendefektstelle (2) zugewandte Seite (10) oder die dem gesunden Knochen (6) zugewandte Seite des Aufsatzes (4)
überragt.

19. Abdeckvorrichtung (1), nach einem der Ansprüche 15 bis 18, **dadurch gekennzeichnet, dass** der Aufsatz (4) an mindestens einer für ein Fixiermittel (5) abgestimmten Fixiermittelposition eine Führung für ein Werkzeug aufweist.

20. Abdeckvorrichtung (1), nach einem der Ansprüche 15 bis 19, **dadurch gekennzeichnet, dass** der Aufsatz (4) aus einem formstabilen Material besteht.

21. Abdeckvorrichtung (1), nach einem der Ansprüche 15 bis 20, **dadurch gekennzeichnet, dass** die dem Knochendefekt zugewandte Seite (10) oder die dem Knochendefekt abgewandte Seite (11) des Aufsatzes (4) zumindest teilweise der Form eines an der Knochendefektstelle (2) regenerierten Knochens entspricht.

22. Abdeckvorrichtung (1), nach einem der Ansprüche 15 bis 21, **dadurch gekennzeichnet, dass** der Aufsatz (4) mindestens eine Öffnung aufweist.

23. Abdeckvorrichtung (1), nach einem der Ansprüche 15 bis 22, **dadurch gekennzeichnet, dass** der Aufsatz (4) mindestens eine Befestigungsvorrichtung für mindestens ein zu setzendes Implantat aufweist.

24. Abdeckvorrichtung (1), nach Anspruch 23, **dadurch gekennzeichnet, dass** mindestens eine Befestigungsvorrichtung durch einen Teil des Aufsatzes (4), der mittels mindestens einer Sollbruchstelle mit dem restlichen Teil des Aufsatzes (4) verbunden ist, zumindest teilweise abgedeckt ist.

25. Abdeckvorrichtung (1), nach einem der Ansprüche 15 bis 24, **dadurch gekennzeichnet, dass** zur Positionierung des Aufsatzes (4) an einem an die Knochendefektstelle (2) angrenzenden gesunden Knochen (6) an dem Aufsatz (4) mindestens ein Positioniermittel (15) angeordnet ist, das eine dem gesunden Knochen abgewandte Seite (16) und eine dem gesunden Knochen (6) und mit diesem zumindest teilweise korrespondierende zugewandte Seite aufweist.

26. Abdeckvorrichtung (1), nach Anspruch 25, **dadurch gekennzeichnet, dass** zwischen dem Aufsatz (4) und mindestens einem Positioniermittel (15) mindestens eine Sollbruchstelle angeordnet ist.

27. Abdeckvorrichtung (1), nach einem der Ansprüche 15 bis 26, **dadurch gekennzeichnet, dass** die Abdeckvorrichtung (1) gemäß dem Verfahren zur Herstellung einer Abdeckvorrichtung für eine Knochendefektstelle (2) nach einem der Ansprüche 1 bis 14 hergestellt wird.

## Claims

1. A method for producing a covering device for a bone defect site (2) which has a top attachment (4) and at least one fixing means (5) for said top attachment (4), the method consisting of the following process steps:
- recording a first data set which represents the involved bone defect site (2) in its current state,
- comparing said first data set with a second data set which represents the target state of regenerated bone at the bone defect site (2),
- utilising said first data set and said second data set for the purpose of planning the top attachment (4),
- which is appropriate for covering at least partially the bone defect site (2), wherein the top attachment (4) has a side (11) facing away from the bone defect site (2) and a side (10) facing towards the bone defect site (2) and wherein the top attachment (4) is suitable for being fixed within the bone defect site (2) by at least one fixing means (5),
or
- which is appropriate for covering at least partially the bone defect site (2) and, in addition, for covering at least partially healthy bone (6) adjoining the bone defect site (2), wherein the top attachment (4) has a side facing away from the healthy bone (6) and a side (11) facing away from the bone defect site (2), as well as a side facing towards the healthy bone (6) and a side (10) facing towards the bone defect site (2), and wherein the top attachment (4) is suitable for being fixed within the bone defect site (2) by at least one fixing means (5) and/or for being fixed onto the healthy bone (6) by at least one fixing means (5),
- implementing the planning of the top attachment (4) in the form of a planning data set, and
- supplying said planning data set to a manufacturing process, in particular to a computer-controlled manufacturing process, wherein concurrently with, and/or subsequent to, the manufacture of the top-structure (4) at least one fixing means (5) suitable for fixing the top attachment (4) within the bone defect site (2) or onto the healthy bone (6) adjoining said bone defect site (2) is detachably connected with the top attachment (4) by means of at least one connection (12) disposed between said fixing means (5) and the top attachment (4), wherein detaching of at least one connection (12) is irreversible, **characterised in that** at least one connection (12) is in the form of a predetermined breaking point (13).

2. The method as claimed in claim 1, **characterised in that** in the course of at least one preceding process step, a fixing means position for at least one fixing means (5) is predefined which is specifically adapted to the utilisation of said fixing means (5) and which enables the top attachment (4) to be fixed within the bone defect site (2) or on the healthy bone (6) in order to obtain, in the course of the manufacturing process, a top attachment (4) in which at least one fixing means (5) in its fixing means position is detachably connected with the top attachment (4).

3. The method as claimed in claim 2, **characterised in that** in the course of the manufacturing process, a guide mechanism for a tool is arranged at a fixing means position adapted for a fixing means (5) which tool permits to drill a hole within the bone defect site (2) or into the healthy bone (6) that is suitable for the fixing means (5).

4. The method as claimed in any of the preceding claims, **characterised in that** the side (10) of the top attachment (4) formed during the manufacturing process which faces towards the bone defect or the side (11) thereof facing away from the bone defect corresponds at least partially to the shape of the regenerated bone in its target state.

5. The method as claimed in any of the preceding claims, **characterised in that** the second data set is either obtained by calculation or was recorded at a time when the bone at the bone defect site (2) which is currently to be regenerated had still been healthy bone (6).

6. The method as claimed in any of the preceding claims, **characterised in that** the recording of the first data set represents the involved bone defect site (2) in its three-dimensionality and/or the recording of the second data set represents the shape of the still healthy bone (6) in its three-dimensionality.

7. The method as claimed in any of the preceding claims, **characterised in that** the recording of the first data set and/or the recording of the second data set is/are obtained by at least one imaging technique.

8. The method as claimed in any of the preceding claims, **characterised in that** the recording of the first data set and/or the recording of the second data set is/are obtained by employing a method permitting three-dimensional bone representation.

9. The method as claimed in any of the preceding claims, **characterised in that** in the course of the manufacturing process, the top attachment (4) is realised by means of 3D printing and/or by means of milling.

10. The method as claimed in any of the preceding claims, **characterised in that** during the manufacture of the top attachment (4) at least one fastening device for at least one implant to be inserted and/or at least one predetermined breaking point (13, 17) and/or at least one positioning means (15) are arranged on said top attachment (4).

11. The method as claimed in claim 10, **characterised in that** at least one fastening device is exposed by removing a part of the top attachment (4) which prior to its removal is connected to the remaining part of the top attachment (4) by means of at least one predetermined breaking point (17).

12. The method as claimed in claim 10 or in claim 11, **characterised in that** at least one positioning means (15) which is arranged on the top attachment (4) during the manufacture of said top attachment (4) serves for positioning the top attachment (4) in an insertion position.

13. The method as claimed in any one of claims 10 to 12, **characterised in that** between the top attachment (4) and at least one positioning means (15) at least one predetermined breaking point (17) is arranged and/or **in that** between the top attachment (4) and at least one fixing means (5) that is arranged on said top attachment (4) at least one predetermined breaking point (13) is arranged.

14. The method as claimed in any of the preceding claims, **characterised in that** the top attachment (4) and/or at least one fixing means (5) and/or at least one positioning means (15) is/are made of a dimensionally stable material.

15. A covering device (1) for a bone defect site (2),
- including a top attachment (4),
- which is suitable for at least partially covering the bone defect site (2), wherein the top attachment (4) has a side (11) facing away from the bone defect site (2) and a side (10) facing towards the bone defect site (2),
or
- which is suitable for at least partially covering the bone defect site (2) and, in addition, for at least partially covering healthy bone (6) adjoining said bone defect site (2), wherein the top attachment (4) has a side facing away from the healthy bone (6) and a side (11) facing away from the bone defect site (2), as well as a side facing towards the healthy bone (6) and a side (10) facing towards the bone defect site (2),
- including at least one fixing means (5) for fixing the top attachment (4) within the bone defect site (2) and/or including at least one fixing means (5) for fixing the top attachment (4) onto the healthy bone (6) adjoining said bone defect site (2),
**characterised in that**
at least one fixing means (5) suitable for fixing the top attachment (4) within the bone defect site (2) or onto healthy bone (6) adjoining said bone defect site (2) is detachably arranged on the top attachment (4) by means of at least one connection (12) disposed between said fixing means (5) and the top attachment (4), wherein detaching of at least one connection (12) is irreversible, **characterised in that** at least one connection (12) is in the form of a predetermined breaking point (13).

16. The covering device (1) as claimed in claim 15, **characterised in that** on the top attachment (4), a fixing means position is present which is designed for a fixing means (5) and specifically adapted to the utilisation of this fixing means (5), enabling the top attachment (4) to be fixed within the bone defect site (2) or on the healthy bone (6), or **in that** on the top attachment (4), at least one fixing means position is present which is designed for at least one fixing means (5) and specifically adapted to the utilisation of at least one fixing means (5), enabling the top attachment (4) to be fixed within the bone defect site (2) or on the healthy bone (6), wherein at least one fixing means (5) is detachably connected to the top attachment (4) in its fixing means position which is specifically adapted to the utilisation of this fixing means (5).

17. The covering device (1) as claimed in claim 15 or claim (16), **characterised in that** the top attachment (4) has at least one predetermined breaking point (17).

18. The covering device (1) as claimed in any one of claims 15 to 17, **characterised in that** at least one fixing means (5)
- protrudes from the side (11) facing away from the bone defect site (2) or from the side of the top attachment (4) facing away from the healthy bone (6)
and/or
- protrudes from the side (10) facing towards the bone defect site (2) or from the side of the top attachment (4) facing towards the healthy bone (6).

19. The covering device (1) as claimed in any one of claims 15 to 18, **characterised in that** the top attachment (4) has a guide mechanism for a tool which is provided in at least one fixing means position adapted for a fixing means (5).

20. The covering device (1) as claimed in any one of claims 15 to 19, **characterised in that** the top attachment (4) is made of a dimensionally stable material.

21. The covering device (1) as claimed in any one of claims 15 to 20, **characterised in that** the side (10) of the top attachment (4) which faces towards the bone defect or the side (11) thereof which faces away from the bone defect corresponds at least partially to the shape of regenerated bone formed at the bone defect site (2).

22. The covering device (1) as claimed in any one of claims 15 to 21, **characterised in that** the top attachment (4) has at least one opening.

23. The covering device (1) as claimed in any one of claims 15 to 22, **characterised in that** the top attachment (4) has at least one fastening device for at least one implant to be inserted.

24. The covering device (1) as claimed in claim 23, **characterised in that** at least one fastening device is at least partially covered by a part of the top attachment (4) which is connected to the remaining part of the top attachment (4) by means of at least one predetermined breaking point.

25. The covering device (1) as claimed in any one of claims 15 to 24, **characterised in that** for the purpose of positioning the top attachment (4) on the healthy bone (6) adjoining the bone defect site (2), said top attachment (4) has at least one positioning means (15) arranged thereon which has a side (16) facing away from the healthy bone and a side facing towards, and corresponding at least partially with, the healthy bone (6).

26. The covering device (1) as claimed in claim 25, **characterised in that** the top attachment (4) and at least one positioning means (15) have at least one predetermined breaking point arranged therebetween.

27. The covering device (1) as claimed in any one of claims 15 to 26, **characterised in that** the covering device (1) is manufactured according to a method for producing a covering device for a bone defect site (2) as claimed in any one of claims 1 to 14.

## Revendications

1. Procédé destiné à fabriquer un dispositif de recouvrement pour un défaut osseux (2) qui présente un support (4) et au moins un moyen de fixation (5) pour ledit support (4), le procédé comprenant les étapes suivantes :
- la collecte d'un premier ensemble de données qui représente le défaut osseux (2) concerné à l'état initial,
- la comparaison du premier ensemble de données avec un deuxième ensemble de données qui représente l'état final visé d'un os régénéré au niveau du défaut osseux (2),
- l'utilisation du premier ensemble de données et du deuxième ensemble de données en vue de concevoir le support (4)
- qui est propre à recouvrir au moins partiellement le défaut osseux (2), le support (4) présentant une face (11) opposée au défaut osseux (2) et une face (10) tournée vers le défaut osseux (2), et le support (4) pouvant être fixé à l'intérieur du défaut osseux (2) à l'aide d'au moins un moyen de fixation (5),
ou
- qui est propre à recouvrir au moins partiellement le défaut osseux (2) et à recouvrir en outre au moins partiellement un os sain (6) contigu au défaut osseux (2), le support (4) présentant une face opposée à l'os sain (6) et une face (11) opposée au défaut osseux (2) ainsi qu'une face tournée vers l'os sain (6) et une face (10) tournée vers le défaut osseux (2), et le support (4) pouvant être fixé à l'intérieur du défaut osseux (2) à l'aide d'au moins un moyen de fixation (5) et/ou pouvant être fixé sur l'os sain (6) à l'aide d'au moins un moyen de fixation (5),
- la réalisation de la conception du support (4) dans un ensemble de données de conception et
- l'importation de l'ensemble de données de conception dans un procédé de fabrication, en particulier un procédé de fabrication assisté par ordinateur, au moins un moyen de fixation (5) propre à fixer le support (4) à l'intérieur du défaut osseux (2) ou sur l'os sain (6) contigu au défaut osseux (2) étant relié, pendant et/ou après la fabrication du support (4), de manière amovible au support (4) au moyen d'au moins une liaison (12) disposée entre le moyen de fixation (5) et le support (4), la suppression d'au moins une liaison (12) étant irréversible, **caractérisé en ce qu'**au moins une liaison (12) est un point destiné à la rupture (13).

2. Procédé selon la revendication 1, **caractérisé en ce que** pendant au moins une étape précédente du procédé est prédéfinie pour au moins un moyen de fixation (5) une position de moyen de fixation spécialement adaptée à l'utilisation dudit moyen de fixation (5) laquelle rend possible une fixation du support (4) à l'intérieur du défaut osseux (2) ou sur l'os sain (6) afin d'obtenir, grâce audit procédé de fabrication, un support (4) dans lequel au moins un moyen de fixation (5) se trouvant dans sa position de moyen de fixation est relié de manière amovible audit support (4).

3. Procédé selon la revendication 2, **caractérisé en ce que** dans ledit procédé de fabrication est disposé, dans au moins une position de moyen de fixation appropriée à un moyen de fixation (5), un guide pour un outil grâce auquel il est possible de réaliser, à l'intérieur du défaut osseux (2) ou sur l'os sain (6), un trou adapté au moyen de fixation (5).

4. Procédé selon l'une quelconque des revendications précédentes, **caractérisé en ce que** la face (10) tournée vers le défaut osseux ou la face (11) opposée au défaut osseux du support (4) réalisé lors du procédé de fabrication correspond au moins partiellement à la forme de l'os régénéré à l'état final visé.

5. Procédé selon l'une quelconque des revendications précédentes, **caractérisé en ce que** le deuxième ensemble de données est obtenu par calcul ou a été collecté à un moment où l'os au niveau du défaut osseux (2) qui doit être maintenant régénéré était encore un os sain (6).

6. Procédé selon l'une quelconque des revendications précédentes, **caractérisé en ce que** la collecte du premier ensemble de données représente le défaut osseux (2) concerné dans sa tridimensionnalité et/ou la collecte du deuxième ensemble de données représente la forme de l'os encore sain (6) dans sa tridimensionnalité.

7. Procédé selon l'une quelconque des revendications précédentes, **caractérisé en ce que** la collecte du premier ensemble de données et/ou la collecte du deuxième ensemble de données est obtenue par au moins un procédé d'imagerie.

8. Procédé selon l'une quelconque des revendications précédentes, **caractérisé en ce que** la collecte du premier ensemble de données et/ou la collecte du deuxième ensemble de données est obtenue par au moins un procédé qui rend possible une représentation tridimensionnelle d'un os.

9. Procédé selon l'une quelconque des revendications précédentes, **caractérisé en ce que** dans ledit procédé de fabrication, le support (4) est réalisé par impression 3D et/ou par fraisage.

10. Procédé selon l'une quelconque des revendications précédentes, **caractérisé en ce que** pendant la fabrication du support (4), au moins un dispositif de fixation pour au moins un implant à poser et/ou au moins un point destiné à la rupture (13, 17) et/ou au moins un moyen de positionnement (15) sont disposés sur le support (4).

11. Procédé selon la revendication 10, **caractérisé en ce qu'**au moins un dispositif de fixation est libéré lorsque l'on retire une partie du support (4) qui, avant d'être retirée, est reliée à la partie restante du support (4) par au moins un point destiné à la rupture (17).

12. Procédé selon la revendication 10 ou selon la revendication 11, **caractérisé en ce qu'**au moins un moyen de positionnement (15) disposé sur le support (4) pendant la fabrication dudit support (4) sert à positionner le support (4) dans une position d'insertion.

13. Procédé selon l'une quelconque des revendications 10 à 12, **caractérisé en ce qu'**au moins un point destiné à la rupture (17) est disposé entre le support (4) et au moins un moyen de positionnement (15) et/ou **en ce qu'**au moins un point destiné à la rupture (13) est disposé entre le support (4) et au moins un moyen de fixation (5) disposé sur ledit support (4).

14. Procédé selon l'une quelconque des revendications précédentes, **caractérisé en ce que** le support (4) et/ou au moins un moyen de fixation (5) et/ou au moins un moyen de positionnement (15) sont réalisés en un matériau à forme stable.

15. Dispositif de recouvrement (1) pour un défaut osseux (2),
- pourvu d'un support (4),
- grâce auquel le défaut osseux (2) peut être au moins partiellement recouvert, le support (4) présentant une face (11) opposée au défaut osseux (2) et une face (10) tournée vers le défaut osseux (2),
ou
- grâce auquel le défaut osseux (2) peut être au moins partiellement recouvert et un os sain (6) contigu au défaut osseux (2) peut être en outre au moins partiellement recouvert, le support (4) présentant une face opposée à l'os sain (6) et une face (11) opposée au défaut osseux (2) ainsi qu'une face tournée vers l'os sain (6) et une face (10) tournée vers le défaut osseux (2),
- pourvu d'au moins un moyen de fixation (5) destiné à fixer le support (4) à l'intérieur du défaut osseux (2) et/ou pourvu d'au moins un moyen de fixation (5) destiné à fixer le support (4) sur l'os sain (6) contigu au défaut osseux (2),
**caractérisé en ce que**
au moins un moyen de fixation (5) propre à fixer le support (4) à l'intérieur du défaut osseux (2) ou sur l'os sain (6) contigu au défaut osseux (2) est disposé de manière amovible sur le support (4) au moyen d'au moins une liaison (12) disposée entre le moyen de fixation (5) et le support (4), la suppression d'au moins une liaison (12) étant irréversible, **caractérisé en ce qu'**au moins une liaison (12) est un point destiné à la rupture (13).

16. Dispositif de recouvrement (1) selon la revendication 15, **caractérisé en ce qu'**il existe sur le support (4), pour un moyen de fixation (5), une position de moyen de fixation spécialement adaptée à l'utilisation dudit moyen de fixation (5) laquelle rend possible une fixation du support (4) à l'intérieur du défaut osseux (2) ou sur l'os sain (6), ou **en ce qu'**il existe sur le support (4), pour au moins un moyen de fixation (5), au moins une position de moyen de fixation spécialement adaptée à l'utilisation d'au moins un moyen de fixation (5) laquelle rend possible une fixation du support (4) à l'intérieur du défaut osseux (2) ou sur l'os sain (6), au moins un moyen de fixation (5) se trouvant dans sa position de moyen de fixation spécialement adaptée à l'utilisation dudit moyen de fixation (5) étant relié de manière amovible au support (4).

17. Dispositif de recouvrement (1) selon la revendication 15 ou selon la revendication 16, **caractérisé en ce que** le support (4) présente au moins un point destiné à la rupture (17).

18. Dispositif de recouvrement (1) selon l'une quelconque des revendications 15 à 17, **caractérisé en ce qu'**au moins un moyen de fixation (5) dépasse
- de la face (11) du support (4) opposée au défaut osseux (2) ou de la face dudit support opposée à l'os sain (6) et/ou
- de la face (10) du support (4) tournée vers le défaut osseux (2) ou de la face dudit support tournée vers l'os sain (6).

19. Dispositif de recouvrement (1) selon l'une quelconque des revendications 15 à 18, **caractérisé en ce que** le support (4) présente un guide pour un outil dans au moins une position de moyen de fixation adaptée à un moyen de fixation (5).

20. Dispositif de recouvrement (1) selon l'une quelconque des revendications 15 à 19, **caractérisé en ce que** le support (4) est constitué en un matériau à forme stable.

21. Dispositif de recouvrement (1) selon l'une quelconque des revendications 15 à 20, **caractérisé en ce que** la face (10) du support (4) tournée vers le défaut osseux ou la face (11) dudit support opposée au défaut osseux correspond au moins partiellement à la forme d'un os régénéré au niveau du défaut osseux (2).

22. Dispositif de recouvrement (1) selon l'une quelconque des revendications 15 à 21, **caractérisé en ce que** le support (4) présente au moins une ouverture.

23. Dispositif de recouvrement (1) selon l'une quelconque des revendications 15 à 22, **caractérisé en ce que** le support (4) présente au moins un dispositif de fixation pour au moins un implant à poser.

24. Dispositif de recouvrement (1) selon la revendication 23, **caractérisé en ce qu'**au moins un dispositif de fixation est recouvert au moins partiellement par une partie du support (4) qui est reliée à la partie restante dudit support (4) par au moins un point destiné à la rupture.

25. Dispositif de recouvrement (1) selon l'une quelconque des revendications 15 à 24, **caractérisé en ce qu'**en vue de positionner le support (4) sur un os sain (6) contigu au défaut osseux (2) est disposé sur ledit support (4) au moins un moyen de positionnement (15) qui présente une face (16) opposée à l'os sain ainsi qu'une une face tournée vers l'os sain (6) et correspondant au moins partiellement à celui-ci.

26. Dispositif de recouvrement (1) selon la revendication 25, **caractérisé en ce qu'**au moins un point destiné à la rupture est disposé entre le support (4) et au moins un moyen de positionnement (15).

27. Dispositif de recouvrement (1) selon l'une quelconque des revendications 15 à 26, **caractérisé en ce que** ledit dispositif de recouvrement (1) est fabriqué conformément au procédé destiné à fabriquer un dispositif de recouvrement pour un défaut osseux (2) selon l'une quelconque des revendications 1 à 14.
